# EUROPEAN PATENT SPECIFICATION

(11) **EP 3 934 800 B1**
(45) Date of publication and mention of the grant of the patent: **29.04.2026**
(21) Application number: 20766866.6
(22) Date of filing: 21.01.2020
(51) Int. Cl.: B01J 13/02, C09D 5/14, A01N 25/28, A01P 1/00, B01J 13/06

(54) **A COMPOSITE AND A METHOD OF PREPARING THE SAME**
VERBUNDMATERIAL UND VERFAHREN ZUR HERSTELLUNG DAVON
COMPOSITE ET PROCÉDÉ DE PRÉPARATION DE CELUI-CI

(30) Priority: 07.03.2019 SG 10201902068U
(43) Date of publication of application: 12.01.2022
(73) Proprietor: Agency for Science, Technology and Research, Singapore 138632 (SG)
(72) Inventor: ZHANG, Yugen, Singapore 138669 (SG); WANG, Jinquan, Singapore 138669 (SG)
(74) Representative: HGF
(86) International application number: PCT/SG2020/050025
(87) International publication number: WO 2020/180242

(56) References cited:
- WO-A1-2010/093909
- WO-A1-2018/056900
- CN-A- 1 473 553
- CN-A- 104 070 162
- CN-A- 106 683 738
- JP-A- H09 208 401
- US-A- 5 330 792
- US-A1- 2007 128 439
- US-A1- 2008 292 671
- US-A1- 2014 049 354
- US-B1- 6 348 265
- DATABASE WPI Week 201872, Derwent World Patents Index; AN 2018-759185, XP002807671
- DATABASE WPI Week 201623, Derwent World Patents Index; AN 2015-665154, XP002807672
- ZERJAV, G. ET AL.: "Carboxylic Acids as Corrosion Inhibitors for Cu, Zn and Brasses in Simulated Urban Rain", INTERNATIONAL JOURNAL ELECTROCHEMICAL SCIENCE, vol. 9, 2 March 2014 (2014-03-02), pages 2696 - 2715, XP055736407
- JIAN-SHU LU: "Corrosion of titanium in phosphoric acid at 250°C.", TRANSACTIONS OF NONFERROUS METALS SOCIETY OF CHINA, vol. 19, no. 3, 9 June 2009 (2009-06-09), China, pages 552 - 556, XP055736411, DOI: 10.1016/S1003-6326(08)60311-8

## Description

### References to Related Applications

This application claims priority to Singapore application number 10201902068U filed on 7 March 2019.

### Technical Field

The present invention relates to a composite, a method of preparing the same and uses thereof.

### Background Art

Microbial infections and the development of antimicrobial resistance have received attention as one of the most critical issues facing the public health and security. The creation of clean antimicrobial surfaces with long-term stabilities and activities have tremendous applications involving almost all aspects of daily life, such as medical devices, hospital surfaces, textiles, packaging, electrical appliances, marine antifouling, filters and public surfaces.

CN 108 543 429 A discloses silver-metal oxide core-shell anti-microbial nanoparticles.

Inorganic antimicrobial materials, especially semiconductor antimicrobial materials, are less prone to chemical contamination and possess long-term stability. Some metal or metal oxides, such as silver, zinc oxide and titanium oxide particles, have been used as antimicrobial ingredients in various products or in antimicrobial surface coatings. However, these materials have various limitations, such as heavy metal contamination/ toxicity for silver-based materials and low antimicrobial efficacies due to dependence on photo irradiation and uncertain nano-toxicity for zinc oxide and titanium oxide materials. Redox active Zn/ZnO, Zn/ZnS and Zn/ZnSₓO₁₋ₓ composite materials have been developed and possess excellent antimicrobial property. However, these types of inorganic composites have difficulties in formulation and dispersion into various matrices.

Therefore, there is a need to provide a process and a composite material for antimicrobial activity that overcome or ameliorate one or more of the disadvantages mentioned above.

### Summary

The present invention relates to a composite comprising an inorganic metal or alloy core at least partially covered by a protection shell layer comprising a metal-amino acid complex,
wherein the metal of the inorganic metal core is the same as the metal in the metal-amino acid complex of the protection shell layer, and
wherein said metal-amino acid complex is formed from an amino acid selected from the group consisting of arginine, histidine, lysine, phenylalanine, tryptophan, glutamic acid, aspartic acid, tyrosine, levothyroxine, hydroxyproline, γ-aminobutyric acid, selenomethionine and carnitine.

The present invention also relates to a method of preparing a composite comprising an inorganic metal or alloy core at least partially covered by a protection shell layer comprising a metal-amino acid complex, wherein the metal of the inorganic metal core is the same as the metal in the metal-amino acid complex of the protection shell layer, and wherein said metal-amino acid complex is formed from an amino acid selected from the group consisting of arginine, histidine, lysine, phenylalanine, tryptophan, glutamic acid, aspartic acid, tyrosine, levothyroxine, hydroxyproline, γ-aminobutyric acid, selenomethionine and carnitine, the method comprising the step of mixing a metal or alloy with the amino acid, at a temperature in the range of 50 °C to 180 °C for a time duration, optionally in the presence of a solvent.

The present invention also relates to the use of a composite as an antimicrobial agent, wherein said composite comprises an inorganic metal or alloy core at least partially covered by a protection shell layer comprising a metal-amino acid complex, wherein the metal of the inorganic metal core is the same as the metal in the metal-amino acid complex of the protection shell layer, and wherein said metal-amino acid complex is formed from an amino acid selected from the group consisting of arginine, histidine, lysine, phenylalanine, tryptophan, glutamic acid, aspartic acid, tyrosine, levothyroxine, hydroxyproline, γ-aminobutyric acid, selenomethionine and carnitine.

The invention is as disclosed in the appended claims.

The present disclosure also relates to a composite comprising an inorganic metal or alloy core at least partially covered by a protection layer comprising a carboxylate salt, a metal oxide, a metal oxide salt or a metal-amino acid complex. Such a composite is disclosed but not claimed.

Advantageously, the composite may demonstrate an antimicrobial activity in the form of antibacterial and antifungal effects. Thus, the composite may be used on its own as a general antimicrobial agent or antimicrobial surface coating or mixed in other systems as an antimicrobial additive.

Further advantageously, the composite may be highly active and stable and may exhibit long-term activity.

Still further advantageously, the composite may be easily synthesized, enabling scale-up manufacturing. The composite may also be readily formulated and dispersed into various matrices, thus it may be provided in different forms, such as films, plates and particles.

The present disclosure also relates to a method of preparing the composite comprising an inorganic metal or alloy core at least partially covered by a protection layer comprising a carboxylate salt, a metal oxide, or a metal oxide salt or a metal-amino acid complex, comprising the step of mixing a metal or alloy with an acid, at elevated temperature for a time duration, optionally in the presence of a solvent.

Such a method is disclosed but not claimed.

Advantageously, the method of preparing the composite may be applied on various metal or alloy surfaces to generate activated surfaces and impose antimicrobial effect.

The present disclosure also relates to use of a composite as an antimicrobial agent, wherein said composite comprises an inorganic metal or alloy core at least partially covered by a protection layer comprising a carboxylate salt, a metal oxide, a metal oxide salt or a metal-amino acid complex.

Such a use is disclosed but not claimed.

Advantageously, the use of the composite may cause a 5-log reduction of gram-positive bacteria, gram-negative bacteria or fungi population within 18 hours.

The present disclosure also relates to a composite for use in the treatment or prophylaxis of microbial infection, wherein said composite comprises an inorganic metal or alloy core at least partially covered by a protection layer comprising a carboxylate salt, a metal oxide, a metal oxide salt or a metal-amino acid complex. Such a composite is disclosed but not claimed.

The present disclosure also relates to use of a composite in the manufacture of a medicament for the treatment or prophylaxis of microbial infection, wherein said composite comprises an inorganic metal or alloy core at least partially covered by a protection layer comprising a carboxylate salt, a metal oxide, a metal oxide salt or a metal-amino acid complex. Such a use is disclosed but not claimed.

The present disclosure also relates to a method of inhibiting microbial activity or treating a microbial infection or disease comprising administering an antimicrobial composition comprising the composite as disclosed herein to a subject or applying the antimicrobial composition on a surface. Such a method is disclosed but not claimed.

### Definitions

The following words and terms used herein shall have the meaning indicated:
The term "composite" as used herein refers to a material made of two or more constituent components that are of different physical and/or chemical properties such that when combined, the resulting material has characteristics different from the constituent components and the individual components remain separate and distinct within the finished structure.

The term "antimicrobial" as used herein refers to the capability to cause cell inhibition, cell injury, cell death or to control the growth of target bacteria and fungi microorganisms.

The term "additive" as used herein refers to a substance that is added to another substance or product in minor quantities to impart or improve certain desired performance properties.

Unless specified otherwise, the terms "comprising" and "comprise", and grammatical variants thereof, are intended to represent "open" or "inclusive" language such that they include recited elements but also permit inclusion of additional, unrecited elements.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1% of the stated value, and even more typically +/- 0.5% of the stated value.

### Detailed Disclosure of Embodiments

Exemplary, non-limiting embodiments of a composite will now be disclosed.

The composite comprises an inorganic metal or alloy core at least partially covered by a protection layer comprising a carboxylate salt, a metal oxide, a metal oxide salt or a metal-amino acid complex.

The core may comprise a metal which may be selected from the group consisting of Group 2, Group 4, Group 7, Group 8, Group 12 or Group 13 of the Periodic Table of Elements, combinations thereof and alloys thereof.

The metal may be selected from the group consisting of zinc, iron, aluminium, titanium, magnesium, and manganese. The metal may be zinc, iron and titanium.

An acid may be used to form the protection layer. Depending on the type of acid used, this will determine the type of protection layer formed, whether the protection layer is a carboxylate salt, a metal oxide, a metal oxide salt or a metal-amino acid complex. The protection layer may also be a hydrated salt. The acid may be selected from the group consisting of organic acids, inorganic acids, and mixtures thereof. The metal (as defined above) or alloy may be reacted with the acid to form the protection layer. Where the acid is an organic acid, the protection layer is a carboxylate salt or an inorganic/organic complex. The carboxylate salt may be a metal carboxylate salt, where the metal of the metal carboxylate salt is as provided above. Where the acid is an inorganic acid, the protection layer is a metal oxide, metal oxide salt or hydrated metal oxide salt.

The organic acid may be selected from the group consisting of carboxylic acids, amino acids, and mixtures thereof.

The carboxylic acid may be a saturated monocarboxylic acid of the formula CH₃(CH₂)ₙCOOH, wherein n may be an integer from 0 to 20.

The carboxylic acid may be selected from the group consisting of saturated fatty acids, unsaturated fatty acids, aromatic carboxylic acids, dicarboxylic acids, tricarboxylic acids, keto acids, α-hydroxyl acids, and divinylether fatty acids.

The carboxylic acid may be selected from the group consisting of methanoic acid, ethanoic acid, propanoic acid, butanoic acid, 2-methylpropanoic acid, pentanoic acid, 3-methylbutanoic acid, hexanoic acid, heptanoic acid, octanoic acid, nonanoic acid, decanoic acid, undecanoic acid, dodecanoic acid, tridecanoic acid tetradecanoic acid, pentadecanoic acid, hexadecanoic acid, heptadecanoic acid, octadecanoic acid, nonadecanoic acid, eicosanoic acid, myristoleic acid, palmitoleic acid, sapienic acid, oleic acid, elaidic acid, vaccenic acid, linoleic acid, linoelaidic acid, α-linoleic acid, arachidonic acid, oxalic acid, succinic acid, adipic acid, maleic acid, fumaric acid, aldaric acid, citric acid, isocitric acid, pyruvic acid, acetoacetic acid, levulinic acid, benzoic acid, salicyclic acid, phthalic acid, glyceric acid, glycolic acid, lactic acid, tartaric acid, and colneleic acid.

The amino acid may be selected from the group consisting of proteinogenic amino acids, non-proteinogenic amino acids, and mixtures thereof.

The amino acid may be selected from the group consisting of arginine, histidine, lysine, phenylalanine, tryptophan, glutamic acid, aspartic acid, tyrosine, γ-aminobutyric acid, carnitine, levothyroxine, hydroxyproline, and selenomethionine. The amino acid may be histidine.

The inorganic acid may be selected from the group consisting of phosphoric acid, polyphosphoric acids, tungstic acids, vanadic acid, molybdic acid, and heteropolyacids.

The protection layer may be polymeric or non-polymeric.

The protection layer may be porous or non-porous.

The protection layer may be regarded as a shell layer.

The size of the inorganic core may be about 0.01 to about 100 µm, about 0.05 to about 100 µm, about 0.1 to about 100 µm, about 0.5 to about 100 µm, about 1 to about 100 µm, about 5 to about 100 µm, about 10 to about 100 µm, about 50 to about 100 µm, about 0.01 to about 0.05 µm, about 0.01 to about 0.1 µm, about 0.01 to about 0.5 µm, about 0.01 to about 1 µm, about 0.01 to about 5 µm, about 0.01 to about 10 µm, about 0.01 to about 50 µm, about 0.05 to about 0.1 µm, about 0.05 to about 0.5 µm, about 0.05 to about 1 µm, about 0.05 to about 5 µm, about 0.05 to about 10 µm, about 0.05 to about 50 µm, about 0.1 to about 0.5 µm, about 0.1 to about 1 µm, about 0.1 to about 5 µm, about 0.1 to about 10 µm, about 0.1 to about 50 µm, about 0.5 to about 1 µm, about 0.5 to about 5 µm, about 0.5 to about 10 µm, about 0.5 to about 50 µm about 1 to about 5 µm, about 1 to about 10 µm, about 1 to about 50 µm, about 5 to about 10 µm, about 5 to about 50 µm, or about 10 to about 50 µm.

The thickness of the protection layer may be about 5 to about 1000 nm, about 8 to about 1000 nm, about 10 to about 1000 nm, about 20 to about 1000 nm, about 50 to about 1000 nm, about 80 to about 1000 nm, about 100 to about 1000 nm, about 200 to about 1000 nm, about 500 to about 1000 nm, about 800 to about 1000 nm, about 5 to about 8 nm, about 5 to about 10 nm, about 5 to about 20 nm, about 5 to about 50 nm, about 5 to about 80 nm, about 5 to about 100 nm, about 5 to about 200 nm, about 5 to about 500 nm, about 5 to about 800 nm, about 8 to about 10 nm, about 8 to about 20 nm, about 8 to about 50 nm, about 8 to about 80 nm, about 8 to about 100 nm, about 8 to about 200 nm, about 8 to about 500 nm, about 8 to about 800 nm, about 10 to about 20 nm, about 10 to about 50 nm, about 10 to about 80 nm, about 10 to about 100 nm, about 10 to about 200 nm, about 10 to about 500 nm, about 10 to about 800 nm, about 20 to about 50 nm, about 20 to about 80 nm, about 20 to about 100 nm, about 20 to about 200 nm, about 20 to about 500 nm, about 20 to about 800 nm, about 50 to about 80 nm, about 50 to about 100 nm, about 50 to about 200 nm, about 50 to about 500 nm, about 50 to about 800 nm, about 80 to about 100 nm, about 80 to about 200 nm, about 80 to about 500 nm, about 80 to about 800 nm, about 100 to about 200 nm, about 100 to about 500 nm, about 100 to about 800 nm, about 200 to about 500 nm, about 200 to about 800 nm, or about 500 to about 800 nm.

The composite may have a protection layer content of about 0.1 % to about 20 % by weight, 0.5 % to about 20 % by weight, 0.8 % to about 20 % by weight, 1 % to about 20 % by weight, 5 % to about 20 % by weight, 8 % to about 20 % by weight, 10 % to about 20 % by weight, 15 % to about 20 % by weight, 18 % to about 20 % by weight, 0.1 % to about 0.5 % by weight, 0.1 % to about 0.8 % by weight, 0.1 % to about 1 % by weight, 0.1 % to about 5 % by weight, 0.1 % to about 8 % by weight, 0.1 % to about 10 % by weight, 0.1 % to about 15 % by weight, 0.1 % to about 18 % by weight, 0.5 % to about 0.8 % by weight, 0.5 % to about 1 % by weight, 0.5 % to about 5 % by weight, 0.5 % to about 8 % by weight, 0.5 % to about 10 % by weight, 0.5 % to about 15 % by weight, 0.5 % to about 18 % by weight, 0.8 % to about 1 % by weight, 0.8 % to about 5 % by weight, 0.8 % to about 8 % by weight, 0.8 % to about 10 % by weight0.8 % to about 15 % by weight, 0.8 % to about 18 % by weight, 1 % to about 5 % by weight, 1 % to about 8 % by weight, 1 % to about 10 % by weight, 1 % to about 15 % by weight, 1 % to about 18 % by weight, 5 % to about 8 % by weight, 5 % to about 10 % by weight, 5 % to about 15 % by weight, 5 % to about 18 % by weight, 8 % to about 10 % by weight, 8 % to about 15 % by weight, 8 % to about 18 % by weight, 10 % to about 15 % by weight, 10 % to about 18 % by weight, or 15 % to about 18 % by weight, based on the weight of the inorganic core.

The particle size of the composite may be in the range of about 100 nm to 500,000 nm.

The composite may be in different forms, such as films, plates and particles.

Exemplary, non-limiting embodiments of a method of preparing the composite will now be disclosed.

The method of preparing the composite comprising an inorganic metal or alloy core at least partially covered by a protection layer comprising a carboxylate salt, a metal oxide, a metal oxide salt or a metal-amino acid complex, may comprise the step of mixing a metal or alloy with an acid, at elevated temperature for a time duration, optionally in the presence of a solvent.

The method may further comprise the steps of cooling the mixture to room temperature, collecting the composite by filtration and repeated washing of the composite with a suitable solvent.

The solvent may be selected from the group consisting of dimethylformamide (DMF), dimethyl sulfoxide (DMSO), dimethylacetamide (DMAc), N-methyl-2-pyrrolidone (NMP), acetonitrile, acetone, ethanol and mixtures thereof.

The elevated temperature used in the method may be of about 50 °C to about 180 °C, about 70 °C to about 180 °C, about 90 °C to about 180 °C, about 110 °C to about 180 °C, about 130 °C to about 180 °C, about 150 °C to about 180 °C, about 170 °C to about 180 °C, about 50 °C to about 70 °C, about 50 °C to about 90 °C, about 50 °C to about 110 °C, about 50 °C to about 130 °C, about 50 °C to about 150 °C, about 50 °C to about 170 °C, about 70 °C to about 90 °C, about 70 °C to about 110 °C, about 70 °C to about 130 °C, about 70 °C to about 150 °C, about 70 °C to about 170 °C, about 90 °C to about 110 °C, about 90 °C to about 130 °C, about 90 °C to about 150 °C, about 90 °C to about 170 °C, about 110 °C to about 130 °C, about 110 °C to about 150 °C, about 110 °C to about 170 °C, about 130 °C to about 150 °C, about 130 °C to about 170 °C, or about 150 °C to about 170 °C.

The time duration used in the method may be of about 1 to about 50 hours, about 5 to about 50 hours, about 10 to about 50 hours, about 20 to about 50 hours, about 30 to about 50 hours, about 40 to about 50 hours, about 45 to about 50 hours, about 1 to about 5 hours, about 1 to about 10 hours, about 1 to about 20 hours, about 1 to about 30 hours, about 1 to about 40 hours, about 1 to about 45 hours, about 5 to about 10 hours, about 5 to about 20 hours, about 5 to about 30 hours, about 5 to about 40 hours, about 5 to about 45 hours, about 10 to about 20 hours, about 10 to about 30 hours, about 10 to about 40 hours, about 10 to about 45 hours, about 20 to about 30 hours, about 20 to about 40 hours, about 20 to about 45 hours, about 30 to about 40 hours, about 30 to about 45 hours, or about 40 to about 45 hours.

The composite prepared from this method is as described above.

Exemplary, non-limiting embodiments of uses of a composite will now be disclosed.

The composite is as described above. The composite may be used as an additive in a composition or a formulation.

When the composite is used as an additive in a composition or a formulation, the composition or formulation may be non-therapeutic.

The composition or formulation may be selected from the group consisting of antimicrobial plastics, antimicrobial pigments, and antimicrobial textiles.

The concentration of the composite may be in the following ranges:
of about 0.1 % to about 4.0 %, about 0.5 % to about 4.0 %, about 1.0 % to about 4.0 %, about 2.0 % to about 4.0 %, about 2.5 % to about 4.0 %, about 3.0 % to about 4.0 %, about 3.5 % to about 4.0 %, about 0.1 % to about 0.5 %, about 0.1 % to about 1.0 %, about 0.1 % to about 2.0 %, about 0.1 % to about 2.5 %, about 0.1 % to about 3.0 %, about 0.1 % to about 3.5 %, about 0.5 % to about 1.0 %, about 0.5 % to about 2.0 %, about 0.5 % to about 2.5 %, about 0.5 % to about 3.0 %, about 0.5 % to about 3.5 %, about 1.0 % to about 2.0 %, about 1.0 % to about 2.5 %, about 1.0 % to about 3.0 %, about 1.0 % to about 3.5 %, about 2.0 % to about 2.5 %, about 2.0 % to about 3.0 %, about 2.0 % to about 3.5 %, about 2.5 % to about 3.0 %, about 2.5 % to about 3.5 %, or about 3.0 % to about 3.5 % by weight of the plastic, of an antimicrobial plastic;
of about 0.1 % to about 4.0 %, about 0.5 % to about 4.0 %, about 1.0 % to about 4.0 %, about 2.0 % to about 4.0 %, about 2.5 % to about 4.0 %, about 3.0 % to about 4.0 %, about 3.5 % to about 4.0 %, about 0.1 % to about 0.5 %, about 0.1 % to about 1.0 %, about 0.1 % to about 2.0 %, about 0.1 % to about 2.5 %, about 0.1 % to about 3.0 %, about 0.1 % to about 3.5 %, about 0.5 % to about 1.0 %, about 0.5 % to about 2.0 %, about 0.5 % to about 2.5 %, about 0.5 % to about 3.0 %, about 0.5 % to about 3.5 %, about 1.0 % to about 2.0 %, about 1.0 % to about 2.5 %, about 1.0 % to about 3.0 %, about 1.0 % to about 3.5 %, about 2.0 % to about 2.5 %, about 2.0 % to about 3.0 %, about 2.0 % to about 3.5 %, about 2.5 % to about 3.0 %, about 2.5 % to about 3.5 %, or about 3.0 % to about 3.5 % by weight of the pigment, of an antimicrobial pigment; and
of about 0.02 % to about 1.00 %, about 0.05 % to about 1.00 %, about 0.10 % to about 1.00 %, about 0.30 % to about 1.00 %, about 0.50 % to about 1.00 %, about 0.70 % to about 1.00 %, about 0.90 % to about 1.00 %, about 0.02 % to about 0.05 %, about 0.02 % to about 0.10 %, about 0.02 % to about 0.30 %, about 0.02 % to about 0.50 %, about 0.02 % to about 0.70 %, about 0.02 % to about 0.90 %, about 0.05 % to about 0.10 %, about 0.05 % to about 0.30 %, about 0.05 % to about 0.50 %, about 0.05 % to about 0.70 %, about 0.05 % to about 0.90 %, about 0.10 % to about 0.30 %, about 0.10 % to about 0.50 %, about 0.10 % to about 0.70 %, about 0.10 % to about 0.90 %, about 0.30 % to about 0.50 %, about 0.30 % to about 0.70 %, about 0.30 % to about 0.90 %, about 0.50 % to about 0.70 %, about 0.50 % to about 0.90 %, or about 0.70 % to about 0.90 % by weight of the textile, of an antimicrobial textile.

There is also provided use of a composite as an antimicrobial agent wherein said composite comprises an inorganic metal or alloy core at least partially covered by a protection layer comprising a carboxylate salt, a metal oxide, a metal oxide salt or a metal-amino acid complex.

The composite is as described above.

The microorganisms may be selected from the group consisting of *Staphylococcus aureus, Escherichia coli, Pseudomonas aeruginosa, Bacillus subtilius, Klebsiella pneumonia, Cryptococcus neoformans* and *Candida albicans.*

The use of the composite may cause a 5-log reduction of gram-positive bacteria, gram-negative bacteria or fungi population within 18 hours.

There is also provided a composite for use in the treatment or prophylaxis of microbial infection, wherein said composite comprises an inorganic metal or alloy core at least partially covered by a protection layer comprising a carboxylate salt, a metal oxide, a metal oxide salt or a metal-amino acid complex.

There is also provided use of a composite, in the manufacture of a medicament for the treatment or prophylaxis of microbial infection, wherein said composite comprises an inorganic metal or alloy core at least partially covered by a protection layer comprising a carboxylate salt, a metal oxide, a metal oxide salt or a metal-amino acid complex.

The composite is as described above.

The microbial infection may be caused by a microbe selected from the group consisting of *Staphylococcus aureus, Escherichia coli, Pseudomonas aeruginosa, Bacillus subtilius, Klebsiella pneumonia*, *Cryptococcus neoformans* and *Candida albicans.*

Exemplary, non-limiting embodiments of a method of inhibiting microbial activity or treating a microbial infection or disease will now be disclosed.

There is also provided a method of inhibiting microbial activity or treating a microbial infection or disease comprising administering an antimicrobial composition comprising the composite as disclosed herein to a subject or applying the antimicrobial composition on a surface.

### Brief Description of Drawings

The accompanying drawings illustrate a disclosed embodiment and serves to explain the principles of the disclosed embodiment. It is to be understood, however, that the drawings are designed for purposes of illustration only, and not as a definition of the limits of the invention.
**Fig.1**

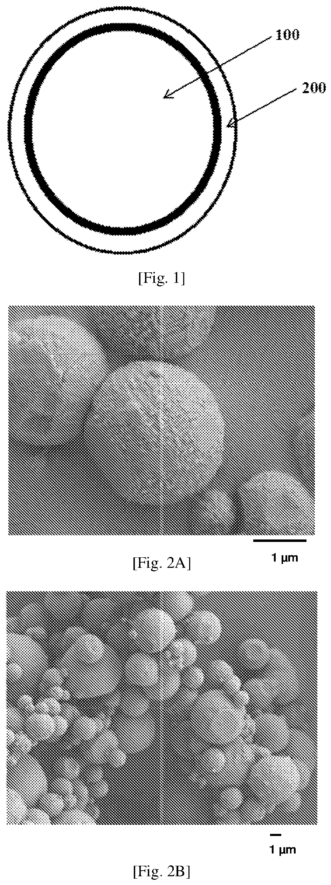

   [Fig. 1] is a schematic illustration of the structure of the composite, where the inorganic metal or alloy core 100 is covered by a protection layer 200. The protection layer also serves to activate the composite towards antimicrobial activity, and enable further formulation and processing of the composite.
**Fig.2A**
   [Fig. 2A] is a scanning electron microscopy (SEM) image of composite A, made in accordance to the synthesis process in Example 1. SEM image of composite A at magnification of x20,000 and scale bar of 1 µm.
**Fig.2B**
   [Fig. 2B] is a SEM image of composite B, made in accordance to the synthesis process in Example 2. SEM image of composite B at magnification of x4,000 and scale bar of 1 µm.
**Fig.2C**

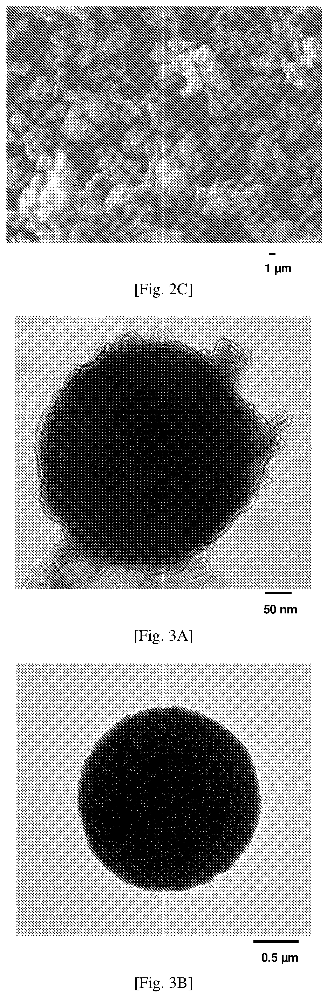

   [Fig. 2C] is a SEM image of composite C, made in accordance to the synthesis process in Example 3. SEM image of composite C at magnification of x3,000 and scale bar of 1 µm.
**Fig.3A**
   [Fig. 3A] is a transmission electron microscopy (TEM) image of composite A, made in accordance to the synthesis process in Example 1. TEM image of composite A at scale bar of 50 nm.
**Fig.3B**
   [Fig. 3B] is a TEM image of composite B, made in accordance to the synthesis process in Example 2. TEM image of composite B at scale bar of 0.5 µm.
**Fig.3C**

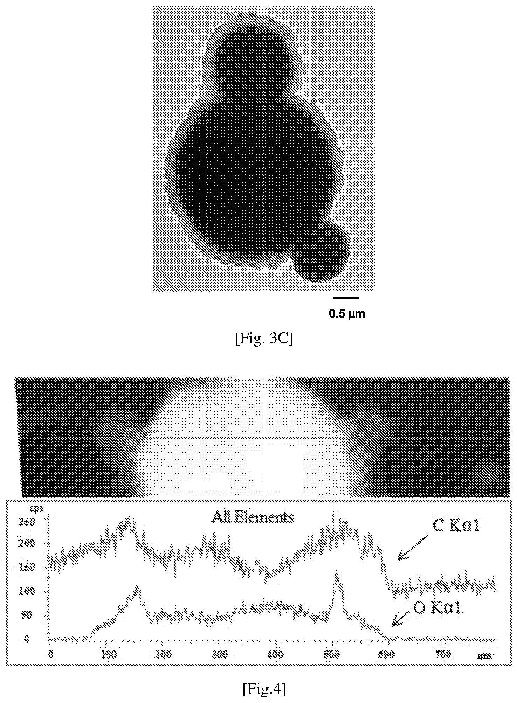

   [Fig. 3C] is a TEM image of composite C, made in accordance to the synthesis process in Example 3. TEM image of composite C at scale bar of 0.5 µm.
**Fig.4**
   [Fig. 4] is a field emission transmission electron microscopy-energy dispersive X-ray (FTEM-EDX) image of composite A, made in accordance to the synthesis process in Example 1, where the scan lines of carbon and oxygen elements over the dimension of composite A are presented.
**Fig.5**

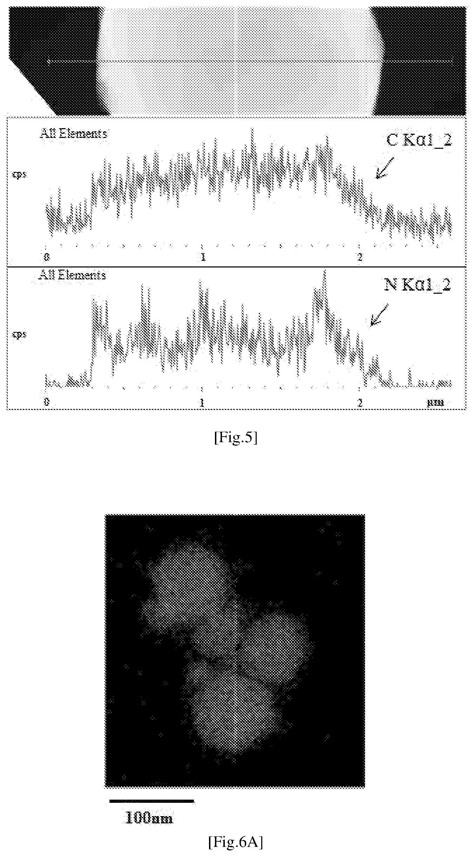

   [Fig. 5] is a FTEM-EDX image of composite B, made in accordance to the synthesis process in Example 2, where the scan lines of carbon and nitrogen elements over the dimension of composite B are presented.
**Fig.6A**
   [Fig. 6A] is a scanning electron microscopy- energy dispersive X-ray (SEM-EDX) elemental mapping image of zinc in composite B (scale bar of 100 nm), made in accordance to the synthesis process in Example 2.
**Fig.6B**

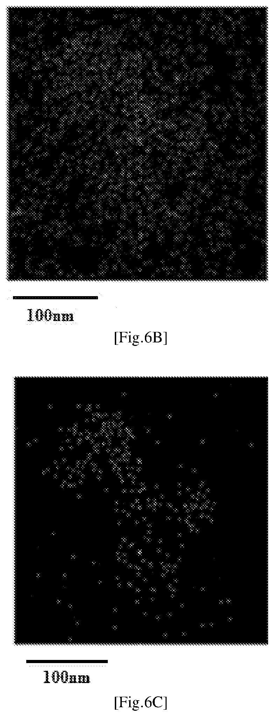

   [Fig. 6B] is a SEM-EDX elemental mapping image of carbon in composite B (scale bar of 100 nm), made in accordance to the synthesis process in Example 2.
**Fig.6C**
   [Fig. 6C] is a SEM-EDX elemental mapping image of nitrogen in composite B (scale bar of 100 nm), made in accordance to the synthesis process in Example 2.
**Fig.6D**

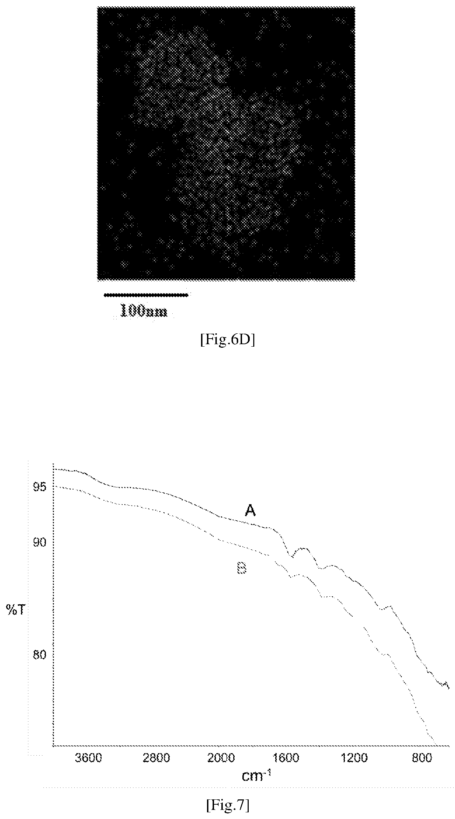

   [Fig. 6D] is a SEM-EDX elemental mapping image of oxygen in composite B (scale bar of 100 nm), made in accordance to the synthesis process in Example 2.
**Fig.7**
   [Fig. 7] is a Fourier-transform infrared spectroscopy (FTIR) of composites A and B made from the synthesis process in Examples 1 and 2 respectively.

### Examples

Non-limiting examples of the invention will be further described in greater detail by reference to specific Examples, which should not be construed as in any way limiting the scope of the invention.

### Materials and Methods

All the reagents were obtained from commercial suppliers and used without further purification. Commercially available zinc powder of 0.4 µm to 10 µm particle size, and titanium powder of 1 µm to 10 µm were purchased from Sigma-Aldrich (of St Louis, Missouri of the United States of America).

The various synthesized composites were subjected to imaging using the scanning electron microscope- energy dispersive X-ray (SEM-EDX) (model JEOL JSM-7400E) at an accelerating voltage of 5 kV. Prior to SEM imaging, the composites were sputter-coated with thin platinum film using the high resolution sputter coater (model JEOL JFC-1600 Auto Fine Coater). The surfaces of the composites were characterized using the transmission electron microscopy (TEM) (model FEI Tecnai F30), field emission transmission electron microscopy-energy dispersive X-ray (FTEM-EDX) (model FEI Tecnai F30), and Fourier-transform infrared spectroscopy (FTIR) (model PerkinElmer Spectrum 100).

Microorganisms (gram-negative bacteria *E. coli* ATCC 8739, gram-positive bacteria *S*. *aureus* ATCC 6538P and *Candida albicans* ATCC-10231) used in the antimicrobial characterization of the synthesized composites were purchased from American Type Culture Collection (ATCC) and re-cultured from lyophilised powders according to suggested protocols. The enrichment medium used for bacteria was a general purpose medium, tryptic soy broth (TSB), purchased from Beckton Dickinson Diagnostics (Singapore). The enrichment medium used for fungi was yeast and malt extract broth (YMB), purchased from Beckton Dickinson Diagnostics (Singapore). TSB and YMB were prepared according to manufacturer's instructions. Prior to microbial experiments, microbial cultures were refreshed on nutrient agar (Millipore, Singapore) plates from stock. Fresh microbial suspensions were grown overnight at 37 °C in 5 ml of TSB or YMB. Microbial cells were collected at the logarithmic stage of growth and the suspensions were adjusted to optical density (OD₆₀₀) of 0.07. Prior to antimicrobial tests, the suspensions were further diluted by 100 times.

### Example 1: Synthesis of carboxylic acid activated zinc powder (composite A) (not according to the invention)

Fresh zinc powder (2 g) was mixed with benzoic acid (0.2 g) in N, N-dimethylformamide (20 ml), and the mixture was stirred at 100 °C for 20 hours. After cooling to room temperature, solid residuals of the composite A were collected by filtration and washed with acetone. The particle size range of the synthesized composite A was measured to be about 400 nm to 1,000 nm.

A schematic representation of the structure of the composite formed is depicted in Fig. 1, where in this case, the zinc is present in the core 100 and the carboxylate salt is present in the protection layer 200. From Fig. 2A, a deposited layer of benzoic acid reacts with the zinc core to form a uniform shell layer of zinc benzoate on the zinc metal particles. The thickness of the protection layer is dependent on parameters such as the quantity of carboxylic acid used and the reaction temperature and duration.

The presence of a protection layer, which was effected by the precursor benzoic acid, was confirmed by Fig. 3A, where a shell was clearly observed covering the zinc particles. The presence of carbon and oxygen elements covering the zinc particle was seen in Fig. 4, where for the region corresponding to the diameter of the zinc particle (between about 150 nm to 500 nm), there were non-zero transmission values for carbon and oxygen elements, indicating the presence of a carboxylate salt covering the zinc core. From Fig. 7, the peaks observed at the fingerprint frequencies along graph A at about 1000 cm⁻¹ indicates the presence of organic groups.

### Example 2: Synthesis of amino acid activated zinc powder (composite B)

Fresh zinc powder (2 g) was mixed with histidine (0.1 g) in ethanol (0.2 ml), and the mixture was stirred at 120 °C for 24 hours. After cooling to room temperature, solid residuals of the composite B were collected. The particle size range of the synthesized composite B was measured to be about 400 nm to 1,000 nm.

A schematic representation of the structure of the composite formed is depicted in Fig. 1, where in this case, the zinc is present in the core 100 and the histidine is present in the form of a zinc-histidine complex in the protection layer 200. From Fig. 2B, a deposited layer of histidine reacts with the zinc core to form a uniform shell layer of zinc-histidine complex on the zinc metal particles. The presence of a protection layer, which was effected by the precursor histidine, was further confirmed by Fig. 3B, where a shell was clearly observed covering the zinc particles. The presence of carbon and nitrogen elements covering the zinc particle was seen in Fig. 5, where for the region corresponding to the diameter of the zinc particle (between about 0.3 µm to 2.3 µm), there were non-zero transmission values for carbon and nitrogen elements, indicating the presence of a zinc-histidine complex covering the zinc core. The presence of carbon, nitrogen and oxygen elements in the shell layer of the composite was further confirmed by SEM-EDX elemental mapping analysis as depicted in Fig. 6A to 6D which show the presence of carbon, nitrogen and oxygen elements respectively. From Fig. 7, the peaks observed at the fingerprint frequencies along graph B at about 1000 cm⁻¹ indicates the presence of organic groups.

### Example 3: Synthesis of inorganic acid activated titanium powder (composite C) (not according to the invention)

Fresh titanium powder (1 g) was mixed with concentrated phosphoric acid (0.03 ml) and the mixture was stirred at 130 °C for 6 hours. After cooling to room temperature, solid residuals of the composite C were collected. The particle size range of the synthesized composite C was measured to be about 400 nm to 1,000 nm.

A schematic representation of the structure of the composite formed is depicted in Fig. 1, where in this case, the titanium is present in the core 100 and the hydrated metal oxide salt is present in the protection layer 200. From Fig. 2C, a deposited layer of phosphoric acid reacts with the titanium core to form a uniform shell layer of titanium oxide phosphate hydrate on the titanium metal particles. The presence of a protection layer, which was effected by the precursor phosphoric acid, was further confirmed by Fig. 3C, where a shell was clearly observed covering the titanium particles.

### Example 4: Antimicrobial properties of the synthesized composites

To test the antimicrobial properties of the synthesized composites, 20 mg synthesized composite was dispersed in ethanol and coated onto a glass slide with a dimension of 2.5 cm x 2.5 cm. An untreated (blank) glass slide was used as a negative control. The antimicrobial properties of the surfaces were evaluated by the JIS Z 2801/ISO 22196 method against *E. coli* (gram-negative, ATCC 8739), *S*. *aureus* (gram-positive, ATCC 6538P) and *C*. *albicans* (fungi). Briefly, 20 mg of synthesized composite was dispersed on a pre-cleaned glass slide and an aliquot of diluted cell suspension (150 µL) (gram positive or gram negative bacteria at concentration of 10⁶ CFU mL⁻¹ or fungi at concentration of 10⁵ CFU mL⁻¹) was used to cover the surface of the glass slide completely. The glass slide was incubated at 37 °C for 18 hours, and the resultant surface was washed and diluted using TSB for bacteria and YMB for fungi, before spreading the washings on two nutrient agar plates. The nutrient agar plates were incubated overnight at 37 °C. The resultant colonies grown on the nutrient agar plates were counted using standard plate counting techniques.

The number of colony forming units (cfu) per mL was calculated and compared against the negative control, to determine the log reduction and the effective killing efficiency of the composites. The number of colony forming units was assumed to be equivalent to the number of viable cells in suspension. The experiment was conducted in triplicates and average results were obtained. After the 18 hours incubation period, microbial growth was observed for the untreated glass slide such that there was an increase of 10² log units from 10⁶ to 10⁸ CFU mL⁻¹, while for glass slides treated with composites A, B and C, there was no observable microbial growth for all three microorganisms tested. Based on log reduction data (Table 1), surfaces coated with composites A, B and C, all showed excellent antimicrobial properties. All tested microbes that were exposed to surfaces with the composites were killed after an 18 hours incubation period such that there were more than 5-log reductions of microbe population observed for *E. coli, S. aureus* and *C*. *albicans,* exhibiting the excellent antimicrobial properties of the synthesized composites.

To test the efficacy of the synthesized composites as additives in imparting antimicrobial properties to substrate materials such as plastics and pigments, polyethylene (PE) plastic and polyacrylic (PA) paint were doped with composite A at 2 % and 1 % by weight of the substrates respectively. Briefly, 10 g PE plastic was melted at about 200 °C and 2 % (w/w) composite A was mixed with molten PE plastic and the resultant mixture was cast into a plate. For polyacrylic paint, commercially available PA paint (without antimicrobial agent) was mixed with 1 % (w/w) composite A to create antimicrobial PA paint. The resultant paint was painted on glass surface and the painted surface was air-dried. Both the PE plastic and painted glass surface which are doped with composite A were evaluated for antimicrobial properties using the JIS Z 2801/ISO 22196 method as described previously.

To test the efficacy of the synthesized composites as additives in antimicrobial textiles, commercially available liquid detergent was mixed with acid-coated composite A at 1 g of composite A for every 150 ml of liquid detergent. The doped detergent liquid was added to textile and washed using the following procedures: 0.01 g composite A was mixed with 1.5 ml commercial liquid detergent and poured into 150 ml water to create a doped washing mixture. The textile (10 g) was added to the doped washing mixture and stirred for 1 hour at a temperature between 25 °C to 60 °C to simulate a washing cycle. After washing, the textile was rinsed with 150m1 of water twice. Thereafter the composite A doped textile was evaluated for antimicrobial properties using the JIS Z 2801/ISO 22196 method as described previously.

Based on log reduction data (Table 1), polyethylene plastic doped with composite A showed excellent antimicrobial properties where more than 5-log reductions of microbial population were observed for *E. coli, S. aureus* and *C*. *albicans.* Polyacrylic paint doped with composite A showed better killing efficacy for *E. coli* (>5-log reductions of population) than *C*. *albicans (>* 2-log reductions of population). Textile doped with composite A showed excellent antimicrobial property where more than 5-log reductions of microbial population were observed for *E. coli.* The results showed that the synthesized composites can be mixed into other systems as additives to impart antimicrobial properties.

**Table 1: Antimicrobial Properties of Different Surfaces Coated with Synthesized Composites**

| Materials | Log Reduction | | |
|---|---|---|---|
| | *E. coli* | *S. aureus* | *C. albicans* |
| Blank glass slide (control) | 0 | 0 | 0 |
| Composite A on glass slide | >5 | >5 | >5 |
| Composite B on glass slide | >5 | >5 | >5 |
| Composite C on glass slide | >5 | >5 | >5 |
| Composite A (2 % by weight) in polyethylene plate | >5 | >5 | >2 |
| Composite A (1 % by weight) in paint | >5 | -- | >2 |
| Composite A (0.05 % by weight) in textile | >5 | -- | -- |

### Industrial Applicability

The composite may be used as a general antimicrobial agent in various applications, such as but not limited to, consumer care, cosmetics, healthcare, personal care, public hygiene, general surface disinfection, antifouling, anti-molding, hospital and medical device disinfection, and agriculture industry.

## Claims

1. A composite comprising an inorganic metal or alloy core at least partially covered by a protection shell layer comprising a metal-amino acid complex,
wherein the metal of the inorganic metal core is the same as the metal in the metal-amino acid complex of the protection shell layer, and
wherein said metal-amino acid complex is formed from an amino acid selected from the group consisting of arginine, histidine, lysine, phenylalanine, tryptophan, glutamic acid, aspartic acid, tyrosine, levothyroxine, hydroxyproline, γ-aminobutyric acid, selenomethionine and carnitine.

2. The composite of claim 1, wherein said metal is selected from the group consisting of Group 2, Group 4, Group 7, Group 8, Group 12 or Group 13 of the Periodic Table of Elements, combinations thereof and alloys thereof.

3. The composite of claim 1 or 2, wherein said metal is selected from the group consisting of zinc, iron, aluminium, titanium, magnesium, and manganese.

4. The composite of any of claims 1 to 3, wherein said protection shell layer is polymeric or non-polymeric; or wherein said protection shell layer is porous or non-porous.

5. The composite of any one of claims 1 to 4, wherein said protection shell layer is in a mass weight percentage range of 0.1 % to 20 % by weight of the inorganic core.

6. A method of preparing a composite comprising an inorganic metal or alloy core at least partially covered by a protection shell layer comprising a metal-amino acid complex,
wherein the metal of the inorganic metal core is the same as the metal in the metal-amino acid complex of the protection shell layer, and
wherein said metal-amino acid complex is formed from an amino acid selected from the group consisting of arginine, histidine, lysine, phenylalanine, tryptophan, glutamic acid, aspartic acid, tyrosine, levothyroxine, hydroxyproline, γ-aminobutyric acid, selenomethionine and carnitine,
the method comprising the step of mixing a metal or alloy with the amino acid, at a temperature in the range of 50 °C to 180 °C for a time duration, optionally in the presence of a solvent.

7. The method according to claim 6, wherein said time duration is in the range of 1 hour to 50 hours.

8. Use of a composite as an antimicrobial agent, wherein said composite comprises an inorganic metal or alloy core at least partially covered by a protection shell layer comprising a metal-amino acid complex,
wherein the metal of the inorganic metal core is the same as the metal in the metal-amino acid complex of the protection shell layer, and
wherein said metal-amino acid complex is formed from an amino acid selected from the group consisting of arginine, histidine, lysine, phenylalanine, tryptophan, glutamic acid, aspartic acid, tyrosine, levothyroxine, hydroxyproline, γ-aminobutyric acid, selenomethionine and carnitine.

9. A composite for use in the treatment or prophylaxis of microbial infection, wherein said composite comprises an inorganic metal or alloy core at least partially covered by a protection shell layer comprising a metal-amino acid complex,
wherein the metal of the inorganic metal core is the same as the metal in the metal-amino acid complex of the protection shell layer, and
wherein said metal-amino acid complex is formed from an amino acid selected from the group consisting of arginine, histidine, lysine, phenylalanine, tryptophan, glutamic acid, aspartic acid, tyrosine, levothyroxine, hydroxyproline, γ-aminobutyric acid, selenomethionine and carnitine.

## Patentansprüche

1. Verbundmaterial, umfassend einen anorganischen Metall- oder Legierungskern, der zumindest teilweise durch eine Schutzhüllenschicht bedeckt ist, die einen Metall-Aminosäure-Komplex umfasst,
wobei das Metall des anorganischen Metallkerns das gleiche wie das Metall in dem Metall-Aminosäure-Komplex der Schutzhüllenschicht ist und
wobei der Metall-Aminosäure-Komplex aus einer Aminosäure ausgebildet ist, die ausgewählt ist aus der Gruppe bestehend aus Arginin, Histidin, Lysin, Phenylalanin, Tryptophan, Glutaminsäure, Asparaginsäure, Tyrosin, Levothyroxin, Hydroxyprolin, γ-Aminobuttersäure, Selenomethionin und Carnitin.

2. Verbundmaterial nach Anspruch 1, wobei das Metall aus der Gruppe ausgewählt ist, die aus Gruppe 2, Gruppe 4, Gruppe 7, Gruppe 8, Gruppe 12 oder Gruppe 13 des Periodensystems der Elemente, Kombinationen davon und Legierungen davon besteht.

3. Verbundmaterial nach Anspruch 1 oder 2, wobei das Metall ausgewählt ist aus der Gruppe bestehend aus Zink, Eisen, Aluminium, Titan, Magnesium und Mangan.

4. Verbundmaterial nach einem der Ansprüche 1 bis 3, wobei die Schutzhüllenschicht polymer oder nicht polymer ist; oder wobei die Schutzhüllenschicht porös oder nicht porös ist.

5. Verbundmaterial nach einem der Ansprüche 1 bis 4, wobei die Schutzhüllenschicht in einem Massengewichtsprozentbereich von 0,1 bis 20 Gew.-% des anorganischen Kerns ist.

6. Verfahren zum Herstellen eines Verbundmaterials, das einen anorganischen Metall- oder Legierungskern umfasst, der zumindest teilweise durch eine Schutzhüllenschicht bedeckt ist, die einen Metall-Aminosäure-Komplex umfasst,
wobei das Metall des anorganischen Metallkerns das gleiche wie das Metall in dem Metall-Aminosäure-Komplex der Schutzhüllenschicht ist und
wobei der Metall-Aminosäure-Komplex aus einer Aminosäure ausgebildet ist, die ausgewählt ist aus der Gruppe bestehend aus Arginin, Histidin, Lysin, Phenylalanin, Tryptophan, Glutaminsäure, Asparaginsäure, Tyrosin, Levothyroxin, Hydroxyprolin, γ-Aminobuttersäure, Selenomethionin und Carnitin,
wobei das Verfahren den Schritt eines Mischens eines Metalls oder einer Legierung mit der Aminosäure bei einer Temperatur im Bereich von 50 °C bis 180 °C für eine Zeitdauer, optional in Gegenwart eines Lösungsmittels, umfasst.

7. Verfahren gemäß Anspruch 6, wobei die Zeitdauer in dem Bereich von 1 Stunde bis 50 Stunden ist.

8. Verwendung eines Verbundmaterials als antimikrobielles Mittel, wobei das Verbundmaterial einen anorganischen Metall- oder Legierungskern umfasst, der zumindest teilweise durch eine Schutzhüllenschicht bedeckt ist, die einen Metall-Aminosäure-Komplex umfasst,
wobei das Metall des anorganischen Metallkerns das gleiche wie das Metall in dem Metall-Aminosäure-Komplex der Schutzhüllenschicht ist und
wobei der Metall-Aminosäure-Komplex aus einer Aminosäure ausgebildet ist, die ausgewählt ist aus der Gruppe bestehend aus Arginin, Histidin, Lysin, Phenylalanin, Tryptophan, Glutaminsäure, Asparaginsäure, Tyrosin, Levothyroxin, Hydroxyprolin, γ-Aminobuttersäure, Selenomethionin und Carnitin.

9. Verbundmaterial zur Verwendung bei der Behandlung oder Prophylaxe einer mikrobiellen Infektion, wobei das Verbundmaterial einen anorganischen Metall- oder Legierungskern umfasst, der zumindest teilweise durch eine Schutzhüllenschicht bedeckt ist, die einen Metall-Aminosäure-Komplex umfasst,
wobei das Metall des anorganischen Metallkerns das gleiche wie das Metall in dem Metall-Aminosäure-Komplex der Schutzhüllenschicht ist und
wobei der Metall-Aminosäure-Komplex aus einer Aminosäure ausgebildet ist, die ausgewählt ist aus der Gruppe bestehend aus Arginin, Histidin, Lysin, Phenylalanin, Tryptophan, Glutaminsäure, Asparaginsäure, Tyrosin, Levothyroxin, Hydroxyprolin, γ-Aminobuttersäure, Selenomethionin und Carnitin.

## Revendications

1. Composite comprenant un noyau en métal ou alliage inorganique au moins partiellement recouvert d'une couche d'enveloppe de protection comprenant un complexe métal-acide aminé, dans lequel le métal du noyau métallique inorganique est le même que le métal dans le complexe métal-acide aminé de la couche d'enveloppe de protection, et
dans lequel ledit complexe métal-acide aminé est formé à partir d'un acide aminé choisi dans le groupe constitué par l'arginine, l'histidine, la lysine, la phénylalanine, le tryptophane, l'acide glutamique, l'acide aspartique, la tyrosine, la lévothyroxine, l'hydroxyproline, l'acide γ-aminobutyrique, la sélénométhionine et la carnitine.

2. Composite de la revendication 1, dans lequel ledit métal est choisi dans le groupe constitué par le groupe 2, le groupe 4, le groupe 7, le groupe 8, le groupe 12 ou le groupe 13 du tableau périodique des éléments, leurs combinaisons et leurs alliages.

3. Composite de la revendication 1 ou 2, dans lequel ledit métal est choisi dans le groupe constitué par le zinc, le fer, l'aluminium, le titane, le magnésium et le manganèse.

4. Composite de l'une quelconque des revendications 1 à 3, dans lequel ladite couche d'enveloppe de protection est polymère ou non polymère ou dans lequel ladite couche d'enveloppe de protection est poreuse ou non poreuse.

5. Composite de l'une quelconque des revendications 1 à 4, dans lequel ladite couche d'enveloppe de protection est dans une plage de pourcentage en poids de 0,1 % à 20 % en poids du noyau inorganique.

6. Procédé de préparation d'un composite comprenant un noyau en métal ou alliage inorganique au moins partiellement recouvert d'une couche d'enveloppe de protection comprenant un complexe métal-acide aminé,
dans lequel le métal du noyau métallique inorganique est le même que le métal dans le complexe métal-acide aminé de la couche d'enveloppe de protection, et
dans lequel ledit complexe métal-acide aminé est formé à partir d'un acide aminé choisi dans le groupe constitué par l'arginine, l'histidine, la lysine, la phénylalanine, le tryptophane, l'acide glutamique, l'acide aspartique, la tyrosine, la lévothyroxine, l'hydroxyproline, l'acide γ-aminobutyrique, la sélénométhionine et la carnitine, le procédé comprenant l'étape de mélange d'un métal ou d'un alliage avec l'acide aminé, à une température comprise dans la plage de 50 °C à 180 °C pendant une durée, éventuellement en présence d'un solvant.

7. Procédé selon la revendication 6, dans lequel ladite durée est comprise dans la plage de 1 heure à 50 heures.

8. Utilisation d'un composite en tant qu'agent antimicrobien, dans laquelle ledit composite comprend un noyau en métal ou alliage inorganique au moins partiellement recouvert d'une couche d'enveloppe de protection comprenant un complexe métal-acide aminé,
dans laquelle le métal du noyau métallique inorganique est le même que le métal dans le complexe métal-acide aminé de la couche d'enveloppe de protection, et
dans laquelle ledit complexe métal-acide aminé est formé à partir d'un acide aminé choisi dans le groupe constitué par l'arginine, l'histidine, la lysine, la phénylalanine, le tryptophane, l'acide glutamique, l'acide aspartique, la tyrosine, la lévothyroxine, l'hydroxyproline, l'acide γ-aminobutyrique, la sélénométhionine et la carnitine.

9. Composite destiné à être utilisé dans le traitement ou la prophylaxie d'une infection microbienne, dans lequel ledit composite comprend un noyau en métal ou alliage inorganique au moins partiellement recouvert d'une couche d'enveloppe de protection comprenant un complexe métal-acide aminé,
dans lequel le métal du noyau métallique inorganique est le même que le métal dans le complexe métal-acide aminé de la couche d'enveloppe de protection, et
dans lequel ledit complexe métal-acide aminé est formé à partir d'un acide aminé choisi dans le groupe constitué par l'arginine, l'histidine, la lysine, la phénylalanine, le tryptophane, l'acide glutamique, l'acide aspartique, la tyrosine, la lévothyroxine, l'hydroxyproline, l'acide γ-aminobutyrique, la sélénométhionine et la carnitine.
